# EUROPÄISCHE PATENTSCHRIFT

(11) **EP 0 575 575 B1**
(45) Veröffentlichungstag und Bekanntmachung des Hinweises auf die Patenterteilung: **11.03.1998**
(21) Anmeldenummer: 93900073.3
(22) Anmeldetag: 04.01.1993
(51) Int. Cl.: B60B 17/00, G01N 3/42

(54) **RADSATZ, VERFAHREN ZU DESSEN HERSTELLUNG UND VORRICHTUNG ZUR DURCHFÜHRUNG DES VERFAHRENS**
WHEEL SET, PROCESS FOR MAKING IT AND DEVICE FOR IMPLEMENTING THE PROCESS
JEU DE ROUES, SON PROCEDE DE FABRICATION ET DISPOSITIF DE MISE EN UVRE DU PROCEDE

(30) Priorität: 14.01.1992 CH 97/92
(43) Veröffentlichungstag der Anmeldung: 29.12.1993
(73) Patentinhaber: FIAT-SIG Schienenfahrzeuge AG, 8212 Neuhausen (CH)
(72) Erfinder: KRETTEK, Ottmar, D-5100 Aachen (DE); MOMBREI, Werner, D-8010 Dresden (DE); HARSY, Gabor, CH-8212 Neuhausen (CH)
(74) Vertreter: Troesch Scheidegger Werner AG
(86) Internationale Anmeldenummer: CH9300001
(87) Internationale Veröffentlichungsnummer: WO9313953

(56) Entgegenhaltungen:
- DE-B- 1 107 694
- US-A- 4 164 141
- US-A- 4 719 793

## Beschreibung

Die vorliegende Erfindung betrifft ein Verfahren zum Herstellen eines Radsatzes, bestehend aus einer Achswelle und zwei Schienenfahrzeugrädern, deren Oberflächenhärte nach der spanenden Bearbeitung am Laufflächenumfang gemessen wird, sowie eine Vorrichtung zum Messen der Oberflächenhärte von Schienenfahrzeugrädern für das Herstellen eines Radsatzes.

Für Schienenfahrzeuge hoher Geschwindigkeit werden die Räder vorwiegend aus unlegierten Kohlenstoffstählen hergestellt und einer Wärmebehandlung unterzogen, die aus einer Normalglühung und anschliessenden Laufkranzvergütung besteht. Eine Ueberprüfung der wärmebehandelten Räder findet lediglich im Rahmen der üblichen Qualitätssicherung statt, z.B. nach dem UIC-Kodex 812-3. Damit ist jedoch nicht sichergestellt, dass praktisch identische Ergebnisse der Wärmebehandlung bei allen Rädern vorliegen. Da bei der anschliessenden Radsatzherstellung die zusammengehörenden Räder zufällig ausgewählt werden, können erhebliche Abweichungen in den Härte- und Festigkeitswerten der Laufflächen eines derartigen Radsatzes auftreten.

Aus diesen Unterschieden zweier zufällig ausgewählter Schienenfahrzeugräder kann daher deren unterschiedliches lokales Verschleissverhalten resultieren, mit der Folge des Unrundwerdens der Räder.

Aus den US-A-4 719 793 und US-A-4 164 141 sind Vorrichtungen bekannt, mit welchen Härtemessungen vorgenommen werden, wobei in beiden Fällen die Härte nach Brinell vermessen wird, d.h. die Härte kann nur durch Ausmessung der Eindrücke erfolgen. Dies schliesst zum vornherein eine Automatisierung aus und wird für den angestrebten Zweck ungeeignet.

Der vorliegenden Erfindung liegt die Aufgabe zugrunde, Radsätze herzustellen, bei denen durch gleichmässiges Verschleissverhalten die Bildung von Unrundheiten vermieden werden.

Dies wird beim eingangs definierten Verfahren erfindungsgemäss durch die Merkmale gemäss dem kennzeichnenden Teil von Anspruch 1 möglich.

Eine Vorrichtung zur Durchführung des Verfahrens ist gekennzeichnet durch den Anspruch 2.

Die Erfindung wird anschliessend beispielsweise anhand von Zeichnungen erläutert.

Die Figuren 1 bis 4 zeigen beispielsweise verschiedene Härtemessvorrichtungen, anhand derer die Erfindung erläutert wird.

Es zeigen:
- Fig. 1: einen Meridianschnitt durch eine Härtemessvorrichtung,
- Fig. 2: einen Ausschnitt aus einer Variante der Vorrichtung nach Fig. 1, in Seitenansicht und rein schematischer Darstellung,
- Fig. 3: eine Aufsicht auf eine Vorrichtung analog denjenigen gemäss Fig. 2,
- Fig. 4: eine Seitenansicht einer weiteren Ausführungsform einer Vorrichtung analog denjenigen nach den Fig. 1 - 3.

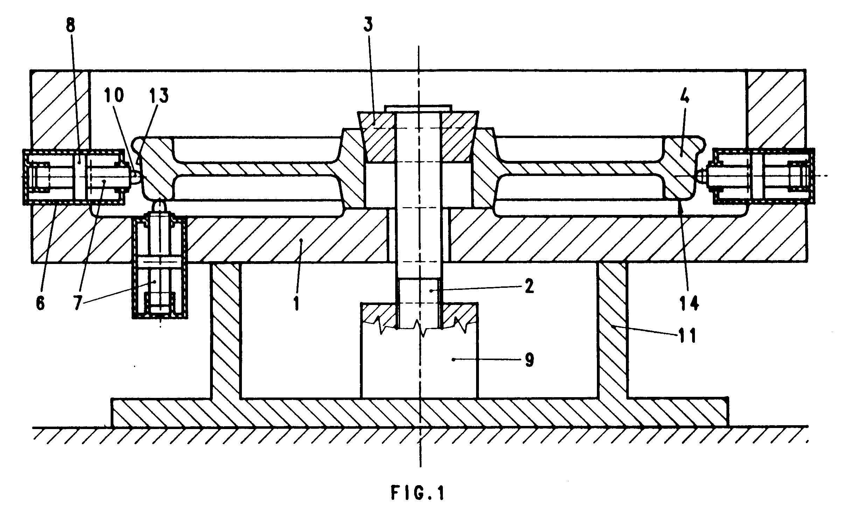

In Fig. 1 ist eine Härte-Mess-Vorrichtung auf einem Untergestell 11 angeordnet. Sie besitzt eine zentrale Spann- und Zentriereinrichtung. Mit ihr wird ein Prüfling 4, ein Schienenfahrzeugrad, in seiner Radnabenbohrung in zentraler Lage aufgenommen und zentriert. Die Spanneinrichtung mit einer Spannwelle 2, einer Zentrierscheibe 3 und einem Spannmotor 9 kann mechanisch, pneumatisch, hydraulisch oder elektrisch betätigt werden. Sie sorgt für einen unverrückbaren Sitz des Prüflings 4 auf einem Prüfring 1. Auf dem äusseren Umfang des Prüfrings 1 sind mehrere, wenigstens jedoch sechs Prüfeinrichtungen 7 horizontal und/oder vertikal in gleichmässigem Abstand zueinander verteilt. Sie sind im vorliegenden Fall über Kolben 8 belastet. Diese Einrichtungen 7 zeichnen nach dem Rockwellverfahren die Eindringtiefe definierter Prüfkörper 10 in den Prüfling 4 auf und ermitteln damit in einem Arbeitsgang die Härte am Laufflächenumfang 13 und/oder an der laufflächennahen Stirnseite 14 des Schienenfahrzeugrades.

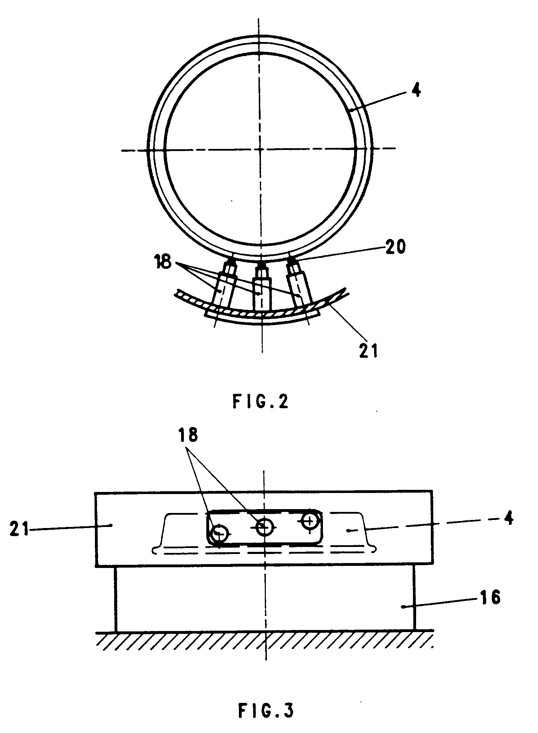

Fig. 2 und 3 zeigt den Prüfling 4 drehbar auf einer zentralen kreisförmigen Grundplatte 16 angeordnet. Am Prüfring 21 sind entweder zwei Prüfeinrichtungen 7 gegenüberliegend angeordnet (Fig. 1) oder drei Prüfeinrichtungen 18, beispielsweise im Abstand von 120° am Umfang des Prüfrings 1 verteilt und mit entsprechenden Prüfkörpern 20 bestückt. Durch Drehen des Prüfrings 21 oder des Prüflings 4 kann in weiteren Arbeitsgängen die Härte anderer Zonen am Laufflächenumfang 13 eines Schienenfahrzeugrades bestimmt werden.

Es ist auch denkbar, die Prüfeinrichtungen 7 bzw. 18 bei den beiden zuvor beschriebenen Vorrichtungen paarweise, seitlich zueinander versetzt (Fig. 3), oder axial verschiebbar im Prüfring 1 bzw. 21 anzuordnen, um so die Härte über die gesamte Laufflächenbreite zu erfassen.

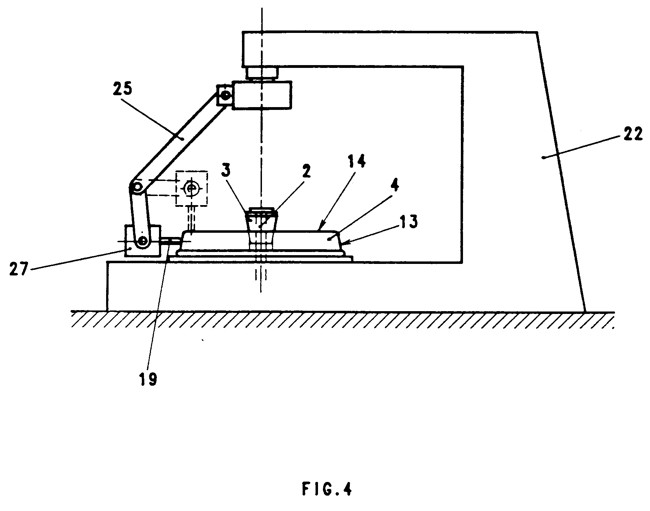

Bei einer in Fig. 4 gezeigten Ausbildungsform der erfindungsgemässen Vorrichtung sind ein oder mehrere Prüfeinrichtungen 27 mit Prüfkörpern 19 an einem, in einer zentralen Achse 2 drehbar an einem an der Vorrichtung 22 angeordneten Gelenkarm 25 vorgesehen, mit dem der Umfang des Prüflings 4 im Laufflächenbereich 13 und/oder an der laufflächennahen Stirnseite 14 des Schienenfahrzeugrades 4 im Bereich von 360° abgetastet werden kann.

Um die üblichen Stückzahlen fertigen zu können, ist vorgesehen, dass die Messung und die Aufzeichnung der Ergebnisse automatisiert erfolgen. Hierzu sind die Prüfkörper 10 bzw. 18 bzw. 19 mittels je eines Anlegemotors mit Kolben 8 und Kolbenführung 6 ausgerüstet. (Fig. 1)

## Patentansprüche

1. Verfahren zum Herstellen eines Radsatzes, bestehend aus einer Achswelle und zwei Schienenfahrzeugrädern, deren Oberflächenhärte nach der spanenden Bearbeitung am Laufflächenumfang (13, 14) gemessen wird, dadurch gekennzeichnet, dass aus einer Mehrzahl von Rädern (4) diejenigen zu einem Schienenfahrzeugradsatz vereinigt werden, deren Oberflächenhärte nach der spanenden Bearbeitung im wesentlichen identische Härte- und Festigkeitswerte aufweisen, wobei die Oberflächenhärte an mindestens sechs am Laufflächenumfang (13, 14) verteilten Stellen ermittelt wird.

2. Vorrichtung zum Messen der Oberflächenhärte von Schienenfahrzeugrädern für das Herstellen eines Radsatzes nach Anspruch 1, dadurch gekennzeichnet, dass am Umfang der Radlauffläche (13, 14) eines drehbar angeordneten Prüflings (4) mehrere an jeweils einem Gelenkarm (25) angeordnete Prüfeinrichtungen gleichmässig positioniert sind.

## Claims

1. Method of manufacturing a wheelset comprising an axle shaft and two rail vehicle wheels, of which the surface hardness after cutting is measured at the running tread periphery (13, 14), characterized in that from a plurality of wheels (4), wheels are selected for combination into a wheelset, which after cutting have a surface hardness presenting substantially identical hardness and strength values, the surface hardness being determined at at least six points distributed over the running tread periphery (13, 14).

2. Apparatus for measuring the surface hardness of rail vehicle wheels for manufacturing a wheelset according to claim 1, characterized in that a plurality of test devices disposed in each case on an articulated arm (25) are uniformly positioned at the periphery of the wheel running tread (13, 14) of a rotatably disposed test sample.

## Revendications

1. Procédé pour fabriquer un jeu de roues comprenant un axe d'essieu et deux roues de véhicules ferroviaires dont la dureté superficielle est mesurée sur la circonférence (13, 14) de la surface de roulement après l'usinage par enlèvement de copeaux, caractérisé en ce que, à partir de plusieurs roues (4), on réunit en un jeu de roues de véhicules ferroviaires celles dont la dureté superficielle après l'usinage par enlèvement de copeaux présente globalement des valeurs de dureté et de résistance identiques, étant précisé que la dureté superficielle est déterminée en au moins six points répartis sur la circonférence (13, 14) de la surface de roulement.

2. Dispositif pour mesurer la dureté superficielle de roues de véhicules ferroviaires pour la fabrication d'un jeu de roues selon la revendication 1, caractérisé en ce que plusieurs dispositifs d'essai sont positionnés de façon régulière sur un bras articulé (25), sur la circonférence (13, 14) de la surface de roulement d'une pièce d'essai (4) mobile en rotation.
